# EUROPÄISCHE PATENTSCHRIFT

(11) **EP 0 581 148 B1**
(45) Veröffentlichungstag und Bekanntmachung des Hinweises auf die Patenterteilung: **15.01.1997**
(21) Anmeldenummer: 93111463.1
(22) Anmeldetag: 16.07.1993
(51) Int. Cl.: C07C 205/12, C07C 201/08

(54) **Verfahren zur Herstellung von 2,3-Dichlor-nitrobenzol**
Process for the preparation of 2,3-dichloro-nitrobenzene
Procédé pour la préparation de 2,3-dichloro-nitrobenzène

(30) Priorität: 29.07.1992 DE 4225023
(43) Veröffentlichungstag der Anmeldung: 02.02.1994
(73) Patentinhaber: BAYER AG, 51368 Leverkusen (DE)
(72) Erfinder: Neumann, Karl Heinz, Dr., D-53757 St. Augustin (DE); Kissener, Wolfram, Dr., D-53819 Neunkirchen/Seelscheid (DE); Fiege, Helmut, Dr., D-51373 Leverkusen (DE)

(56) Entgegenhaltungen:
- WO-A-92/11227
- US-A- 4 453 027
- CHEMICAL ABSTRACTS, vol. 81, no. 15, 14. Oktober 1974, Columbus, Ohio, US; abstract no. 90730n, ZAKHAROV, E.V. ET AL. 'Nitration of o-dichlorobenzene.' Seite 400 ;Spalte 1 ;

## Beschreibung

Die Erfindung betrifft ein Verfahren zur Herstellung von 2,3-Dichlor-nitrobenzol durch Nitrierung von 1,2-Dichlorbenzol unter Verwendung einer wasserfreien Mischsäure aus Salpetersäure, Schwefelsäure und Phosphorsäure als Nitriermedium.

Dichlor-nitrobenzole sind wichtige Zwischenprodukte für die Synthese von Pharmaka und Pflanzenschutzmitteln. Die derzeit bekannten Verfahren zur Nitrierung von 1,2-Dichlorbenzol ergeben eine Mischung aus 2,3-Dichlor-1-nitrobenzol und 3,4-Dichlor-1-nitrobenzol. Die Marktnachfrage nach den beiden Verbindungen unterliegt Veränderungen. So bestand bisher ein größerer Bedarf an dem 3,4-Isomeren. Unter den bisher praktizierten Nitrierbedingungen war die Bildung dieses Isomeren begünstigt, so daß die Produktion der Marktanforderung folgen konnte.

Inzwischen hat sich aber die Marktnachfrage nach dem 2,3-Isomeren erhöht. Um diesen erhöhten Bedarf befriedigen zu zu können, ohne gleichzeitig zusätzliches 3,4-Isomeres herzustellen, ist eine Produktionsweise erforderlich, mit der die Entstehung der beiden Isomeren gesteuert werden kann, mit der insbesondere die Produktion des bisher in starkem Unterschuß anfallenden 2,3-Isomeren gegenüber dem 3,4-Isomeren erhöht werden kann.

Aus der Nitrierung des Monochlorbenzols ist bereits der Einsatz von Phosphorsäure im Nitriersäuregemisch bekannt, um das Verhältnis der beiden Isomeren, nämlich des 2-Chlor- und des 4-Chlor-nitrobenzols zu beeinflussen.

So beschreibt DE-OS 24 22 305 ein Verfahren, in welchem mit 90 %iger Salpetersäure als dem Nitrieragens und in einem Nitriermedium aus Phosphorsäure und Schwefelsäure gearbeitet wird. Mit diesem Verfahren kann das Verhältnis des para- zum ortho-Isomeren von 1,63 auf 1,2 verschoben werden. Die genannte DE-OS' 305 gibt keine Konzentration der Phosphorsäure an; es kann daher vermutet werden, daß handelsübliche, 85 gew`-%ige Phosphorsäure verwendet wird. Ferner gibt die genannte DE-OS' 305 keine Ausbeuten und keine Angaben zur Dinitrierung an. Nach eigenen Untersuchungen betragen die Ausbeuten nach diesem Verfahren nur etwa 80 % der theoretischen Ausbeute.

Ausbeuteangaben (bis zu 93 %) finden sich in DE-OS 24 22 306, die sich offensichtlich als eine Verbesserung des Verfahrens der DE-OS'305 versteht. Hierzu wird ein Metallkatalysator, beispielsweise einer aus Molybdän, Mangan, Vanadin oder Wolfram zum sauren Nitriermedium gegeben.

Ein solches Verfahren bringt jedoch schwerwiegende Entsorgungsprobleme mit sich.

Beide Schriften, nämlich DE-OS'305 und DE-OS'306, enthalten keinerlei Angaben zu einer etwaigen Übertragung der darin beschriebenen Verfahren auf Dichlorbenzole, speziell auf das 1,2-Dichlorbenzol.

Ebenfalls auf die Nitrierung von Monochlorbenzol ist ein anderes Verfahren gerichtet, das in DE-OS 32 44 293 veröffentlicht wurde. Darin wird in einem Nitriermedium, das nur aus Phosphorsäure besteht, mit höchstens einem Mol Salpetersäure pro Mol Chlorbenzol nitriert. Die eingesetzte Phosphorsäure wird in aufkonzentrierter Form mit einem P₂O₅-Gehalt von mehr als 72,4 Gew.-% eingesetzt. Auf Schwefelsäure wird völlig verzichtet. Gemäß Beschreibung dieser DE-OS'293 kann ein para-/ortho-Verhältnis der Chlornitrobenzole von 0,9 erhalten werden. Die Ausbeuten, bezogen auf die eingesetzte Salpetersäure, sind sehr hoch, wenn die Salpetersäure in größerem Unterschuß eingesetzt wird. Dies bedeutet jedoch gleichzeitig eine unvollständige Umsetzung des Chlorbenzols. Ein solch ungenügender Umsatz ist nur bei kostengünstigen Startmaterialien, wie dem Monochlorbenzol, vertretbar und bringt zudem Recyclisierungsprobleme. Auch diese DE-OS'293 gibt keinerlei Hinweise darüber, ob das darin beschriebene Verfahren auf andere Substrate, wie Dichlorbenzole, speziell auf das 1,2-Dichlorbenzol übertragen werden kann.

Die Beschränkung der angegebenen Verfahrensbeschreibungen auf das Monochlorbenzol ist daher verständlich, daß Dichlorbenzole infolge der desaktivierenden Wirkung des zweiten Halogenatoms grundsätzlich reaktionsträge sind, wobei als zusätzliche Schwierigkeit die konkurrierende dirigierende Wirkung zweier Substituenten hinzukommt. Es bestand daher der Bedarf an einem Verfahren zur Herstellung von 2,3-Dichlor-nitrobenzol, das hohe Ausbeuten liefert und zugleich die Möglichkeit bietet, einen Mehrbedarf an 2,3-Dichlor-nitrobenzol zu befriedigen.

Es wurde ein Verfahren zur Herstellung von 2,3-Dichlor-nitrobenzol durch Nitrieren von 1,2-Dichlorbenzol mit Salpetersäure gefunden, das dadurch gekennzeichnet ist, daß zur Nitrierung ein wasserfreies Gemisch aus Phosphorsäure, Schwefelsäure und Salpetersäure eingesetzt wird, wobei eine der Säuren mit einem Anhydridanteil eingesetzt wird, der geeignet ist, den geringen Wasseranteil der beiden anderen Säuren aufzubrauchen.

Nitriert man in bisher bekannter Weise in einem HNO₃/H₂SO₄-Gemisch ohne Zusatz von H₃PO₄, so entstehen bei der Nitrierung von 1,2-Dichlorbenzol die beiden Isomeren typischerweise in etwa 98,5 %iger Ausbeute in einem Verhältnis von 3,4-Dichlor-1-nitrobenzol zu 2,3-Dichlor-1-nitrobenzol von 8,2:1. Nitriert man das 1,2-Dichlorbenzol unter Verwendung von Phosphorsäure, die auf 104 % aufkonzentriert ist, unter gleichzeitigem Verzicht auf Schwefelsäure unter weiterem Einsatz von nahezu wasserfreier Salpetersäure, so erhält man eine Verschiebung zugunsten des 2,3-Isomeren. Das Verhältnis 3,4-:2,3-Dichlor-1-nitrobenzol sinkt demnach auf Werte von 5,95 bzw. 5,45 in geringer Abhängigkeit von der Reaktionstemperatur, was durch eigene Versuche gefunden wurde. Die Bezeichnung 104 %ige H₃PO₄ bezeichnet eine aufkonzentrierte, d.h. entwässerte Phosphorsäure, die einen Anteil Pyrophosphorsäure enthält und demnach einen P₂O₅-Gehalt hat, der 104 % H₃PO₄ entspricht. Der gravierende Nachteil einer solchen Verfahrensweise liegt in dem starken Absinken der Ausbeute auf 89 % bzw. sogar 86 % in gewisser Abhängigkeit von der Reaktionstemperatur.

Es ist nun überraschend, daß bereits ein kleiner Zusatz von Schwefelsäure zum Reaktionsmedium das günstige Isomerenverhältnis beibehält oder sogar noch geringfügig verbessert, aber gleichzeitig die Gesamtausbeute auf Werte weit über 90 %, in vielen Fällen über 95 % der theoretischen Ausbeute bringt.

Das erfindungsgemäße Verfahren ist durch den Einsatz eines wasserfreien Nitriersäuregemisches gekennzeichnet. Ein solches Nitriersäuregemisch wird durch Zusammengeben von technisch verfügbaren, nahezu wasserfreien Säuren hergestellt, wobei eine der Säuren mit einem Anhydridanteil eingesetzt wird, der geeignet ist, den geringen Wasseranteil der beiden anderen Säuren aufzubrauchen. So kann beispielsweise durch Einsatz einer 98 %igen HNO₃ und einer 98 %igen H₂SO₄ gearbeitet werden, wenn die Phosphorsäure eine in der oben beschriebenen Weise aufkonzentrierte Säure ist, die also einen Anteil an Pyrophosphorsäure enthält, was einem Anteil Säureanhydrid P₂O₅ entspricht, der über den Gehalt der 100%igen H₃PO₄ hinausgeht. Es ist jedoch gleichfalls denkbar, daß eine Salpetersäure und eine Phosphorsäure mit einem geringen Wassergehalt eingesetzt werden, wenn gleichzeitig statt der Schwefelsäure Oleum eingesetzt wird, dessen Gehalt an SO₃ geeignet ist, das von den anderen Säuren eingeschleppte Wasser aufzubrauchen. Selbstverständlich ist es weiterhin möglich, eine stärker wasserhaltige HNO₃ einzusetzen, wenn die Phosphorsäure in Form der aufkonzentrierten Säure und/oder die Schwefelsäure in Form von Oleum eingesetzt werden.

In bevorzugter Weise werden die Salpetersäure und die Schwefelsäure als technisch verfügbare etwa 98 %ige Säuren eingesetzt und die Phosphorsäure wird in Form einer aufkonzentrierten Säure eingesetzt. Es ist selbstverständlich, daß Phosphorsäure und/oder Schwefelsäure mit einem Anhydridgehalt in der oben beschriebenen Art auch in einer Menge eingesetzt werden können, die über den Verbrauch des mit der anderen Säure (den anderen Säuren) eingeschleppten Wassers hinausgeht. Auch ein solches Nitriersäuregemisch, das noch einen Anhydridanteil in Form von Pyrophosphorsäure enthält ist ein wasserfreies Säuregemisch im Sinne der vorliegenden Erfindung.

Das molare Mischungsverhältnis von Schwefelsäure und Phosphorsäure nimmt Werte von 0,05 bis 3 Mol H₂SO₄:1 Mol H₃PO₄, bevorzugt 0,1 bis 1,5 Mol H₂SO₄:1 Mol H₃PO₄, ganz besonders bevorzugt von 0,12 bis 0,5 Mol H₂SO₄:1 Mol H₃PO₄ an.

Das erfindungsgemäße Verfahren wird bei einer Reaktionstemperatur von 30 bis 180°C, bevorzugt 60 bis 140°C, besonders bevorzugt 75 bis 125°C durchgeführt. Hierbei wurde gefunden, daß der Anteil des 2,3-Dichlor-nitro-benzols gegenüber 3,4-Dichlor-nitrobenzol bei höherer Temperatur größer ist als bei tieferer Temperatur innerhalb des angegebenen Bereiches. Bei dieser Veränderung der Isomerenzusammensetzung wird die Ausbeute insgesamt nur unwesentlich erniedrigt.

Das molare Verhältnis der Salpetersäure zum Dichlorbenzol beträgt 0,7 bis 1,4 : 1. bevorzugt 0,85 bis 1,3 : 1, ganz besonders bevorzugt 1,0 bis 1,2 : 1.

Nach Beendigung der Nitrierung tritt im allgemeinen eine Phasentrennung in eine obere organische Phase und eine untere Säurephase ein. Nach Abtrennung der organischen Phase wird das darin enthaltene Isomerengemisch 2,3-Dichlor-nitrobenzol/ 3,4-Dichlor-nitrobenzol durch weitere wäßrige Aufarbeitung (Abtrennung restlicher Säure) aufgearbeitet und durch übliche Methoden, wie Destillation, Kristallisation oder Chromatographie in die beiden Isomeren aufgetrennt. Es ist nun eine weitere vorteilhafte Variante des erfindungsgemäßen Verfahrens, daß man die bei dieser Aufarbeitung anfallende untere Säurephase durch Destillation, bevorzugt unter vermindertem Druck, vom Reaktionswasser aus der Nitrierreaktion befreien kann und dadurch erneut eine wasserfreie Mischsäure, bestehend im wesentlichen aus Schwefelsäure und Phosphorsäure herstellen kann. In besonders bevorzugter Weise wird hierbei die Wasserentfernung soweit vorangetrieben, daß das Gemisch wieder Anteile an Anhydrid (z.B. in Form von Pyrophosphorsäure) enthält. Hierauf werden die verbrauchte Salpetersäure und die in der Produktphase verbliebenen geringen Restesäuremengen ergänzt, woraufhin dieses Nitriersäuregemisch erneut einsatzbereit ist.

Für den Fall, daß eine ausgeprägte Trennung in eine organische Produktphase und eine anorganische Mischsäurephase nicht eintritt, kann dies durch Zusatz von wenig Wasser bewirkt werden.

Es ist möglich, das erfindungsgemäße Verfahren absatzweise oder kontinuierlich durchzuführen.

Die nachfolgenden Beispiele erläutern das erfindungsgemäße Verfahren, ohne es auf diese Beispiele einzuschränken.

### Beispiel 1 (zum Vergleich)

1,36 Mol 1,2-Dichlorbenzol wurden in 1,64 Mol 80,5 %iger H₂SO₄ vorgelegt. Bei 45 bis 50°C tropfte man innerhalb von 1,5 h ein Gemisch aus 1,38 Mol 98 %iger HNO₃ und 1,31 Mol 98 %iger H₂SO₄ unter schnellem Rühren zu. Anschließend rührte man bei 45 bis 50°C noch 2 h nach. Die organische Phase wurde warm von der wäßrigen Phase abgetrennt und mit verdünnter Na₂CO₃-Lösung und H₂O neutral gewaschen.

Dieser Versuch zeigt das Ergebnis eines heute in der Technik üblichen Nitrierverfahrens.

| Ausbeute | 3,4-Isomeres | 2,3-Isomeres | Edukt | f = 3,4/2,3 |
|---|---|---|---|---|
| 98 % d.Th. | 88,4 % | 10,8 % | 0,8 % | 8,2 |

### Beispiele 2 bis 3 (zum Vergleich)

Man wiederholte das Verfahren aus Beispiel 1 mit dem Unterschied, daß die 80,5 %ige bzw. 98 %ige H₂SO₄ durch 1,64 Mol bzw. 1,31 Mol auf 104 % (fiktiver H₃PO₄-Gehalt infolge H₂O-Entfernung und Bildung von H₄P₂O₇) aufkonzentrierte H₃PO₄ ersetzt und die Nitriertemperatur auf 85 bis 110°C erhöht wurde.

**Tabelle 1**

| Nr. | T (°C) | Ausbeute | 3,4-Isomeres | 2,3-Isomeres | Edukt | f (3,4/2,3) |
|---|---|---|---|---|---|---|
| 2) | 85 | 89,4 % | 79,01 | 13,28 | 7,71 | 5,95 |
| 3) | 105-110 | 86,1 % | 76,81 | 13,87 | 9,32 | 5,54 |

### Beispiele 4 bis 6

1,36 Mol 1,2-Dichlorbenzol wurden in einem Gemisch aus 0,42 Mol 98 %iger H₂SO₄ und 1,411 Mol auf 104 % aufkonzentrierter H₃PO₄ vorgelegt. Man tropfte unter gutem Rühren bei Reaktionstemperaturen von 85°C bis 125°C ein Nitriersäuregemisch aus 1,38 Mol 98 %iger HNO₃, 0,327 Mol 98 %iger H₂SO₄ und 1,127 Mol 104 %iger H₃PO₄ innerhalb von 1,5 h zu. Man ließ noch 2 h bei 85°C bis 125°C nachrühren und trennte anschließend warm die wäßrige Phase von der organischen Phase ab. Die organische Phase wurde mit verdünnter Na₂CO₃-Lösung und H₂O neutral gewaschen.

**Tabelle 2**

| Nr. | T (°C) | Ausbeute | 3,4-Isomeres | 2,3-Isomeres | Edukt | f (3,4/2,3) |
|---|---|---|---|---|---|---|
| 4) | 85 | 96,6 % | 83,50 | 14,15 | 2,35 | 5,89 |
| 5) | 105-110 | 93,7 % | 80,49 | 14,80 | 4,71 | 5,44 |
| 6) | 125 | 91,9 % | 79,46 | 15,12 | 5,42 | 5,25 |

### Beispiele 7 bis 12

Man legte 1,36 Mol o-Dichlorbenzol in einer Mischung aus 0,38 Mol 98 %iger H₂SO₄ und 1,305 Mol 104 %iger H₃PO₄ vor. Bei einer Temperatur von 105 bis 110°C tropfte man ein Nitriersäuregemisch aus 1,38 Mol 98 %iger HNO₃, 0,38 Mol 98 %iger H₂SO₄ und 1,305 Mol 104 %iger H₃PO₄ innerhalb von 1,5 h unter schnellem Rühren zu. Danach wurde noch weitere 2 h bei gleicher Temperatur gerührt. Die organische Phase wurde warm von der Säurephase abgetrennt und mit verdünnter Na₂CO₃-Lösung und H₂O gewaschen. Die wäßrigen Phasen wurden mit CH₂Cl₂ extrahiert und die vereinigte organische Phase eingeengt.

Die Säurephase wurde dann bei 20 mm Hg-Säule bis zu einer Sumpftemperatur von 180°C andestilliert und anschließend 3 h getempert. Der Verlust bei der aufkonzentrierten Abfallsäure wurde durch Zugabe frischer 104 %iger H₃PO₄ ausgeglichen und die so aufgearbeitete Säure in der nächsten Nitrierung wieder eingesetzt.

**Tabelle 3**

| Nr. | Säurevorlage | zugetropfte Säure | Ausbeute (%) | 3,4-Isomeres | 2,3-Isomeres | Edukt | f (3,4/2,3) |
|---|---|---|---|---|---|---|---|
| 7) | 0,38 Mol H₂SO₄ | 0,38 Mol H₂SO₄ | 94,3 | 81,30 | 14,96 | 3,74 | 5,43 |
| | 1,305 Mol H₃PO₄ | 1,305 Mol H₃PO₄ | | | | | |
| 8) | recycl. | recycl. | 94,8 | 81,39 | 14,91 | 3,70 | 5,46 |
| 9) | recycl. | recycl. | 94,7 | 81,57 | 14,83 | 3,60 | 5,50 |
| 10) | recycl. | recycl. | 95,2 | 81,63 | 14,90 | 3,47 | 5,47 |
| 11) | recycl. | recycl. | 95,4 | 82,14 | 14,92 | 2,94 | 5,50 |
| 12) | recycl. | recycl. | 96,0 | 82,00 | 14,92 | 3,08 | 5,49 |

## Patentansprüche

1. Verfahren zur Herstellung von 2,3-Dichlor-nitrobenzol durch Nitrieren von 1,2-Dichlorbenzol mit Salpetersäure, dadurch gekennzeichnet, daß zur Nitrierung ein wasserfreies Gemisch aus Phosphorsäure, Schwefelsäure und Salpetersäure eingesetzt wird, wobei eine der Säuren mit einem Anhydridanteil eingesetzt wird, der geeigent ist, den geringen Wasseranteil der beiden anderen Säuren aufzubrauchen.

2. Verfahren nach Anspruch 1, dadurch gekennzeichnet, daß neben nahezu wasserfreier Salpetersäure und nahezu wasserfreier Schwefelsäure eine auf über 72,4 Gew.-% P₂O₅ aufkonzentrierte Phosphorsäure eingesetzt wird.

3. Verfahren nach Anspruch 1, dadurch gekennzeichnet, daß das molare Mischungsverhältnis von Schwefelsäure und Phosphorsäure Werte von 0,05 bis 3 Mol H₂SO₄ :1 Mol H₃PO₄ annimmt.

4. Verfahren nach Anspruch 1, dadurch gekennzeichnet, daß die Nitrierung bei einer Reaktionstemperatur von 30 bis 180°C durchgeführt wird.

5. Verfahren nach Anspruch 1, dadurch gekennzeichnet, daß die Salpetersäure in einem molaren Verhältnis von 0,7 bis 1,4 eingesetzt wird.

6. Verfahren nach Anspruch 4, dadurch gekennzeichnet, daß zur Erhöhung des Anteils an 2,3-Dichlor-nitrobenzol gegenüber 3,4-Dichlor-nitrobenzol im oberen Teil des Temperaturbereiches gearbeitet wird.

7. Verfahren nach Anspruch 1, dadurch gekennzeichnet, daß das bei der Produktaufarbeitung erhaltene Säuregemisch durch Destillation vom Wasser befreit und recyclisiert wird.

## Claims

1. Process for the preparation of 2,3-dichioro-nitrobenzene by nitration of 1,2-dichlorobenzene with nitric acid, characterised in that an anhydrous mixture of phosphoric acid, sulphuric acid and nitric acid is employed for the nitration, one of the acids being employed with an anhydride content which is suitable for using up the low water content of the other two acids.

2. Process according to Claim 1, characterised in that, in addition to nearly anhydrous nitric acid and nearly anhydrous sulphuric acid, a phosphoric acid concentrated to over 72.4 % by weight of P₂O₅ is employed.

3. Process according to Claim 1, characterised in that the molar mixing ratio of sulphuric acid and phosphoric acid assumes values from 0.05 to 3 mol of H₂SO₄ : 1 mol of H₃PO₄.

4. Process according to Claim 1, characterised in that the nitration is carried out at a reaction temperature of 30 to 180°C.

5. Process according to Claim 1, characterised in that the nitric acid is employed in a molar ratio of 0.7 to 1.4.

6. Process according to Claim 4, characterised in that, to increase the proportion of 2,3-dichioro-nitrobenzene compared to 3,4-dichloro-nitrobenzene, the reaction is carried out in the upper part of the temperature range.

7. Process according to Claim 1, characterised in that the acid mixture obtained in the product work-up is freed from water by distillation and recycled.

## Revendications

1. Procédé pour la préparation du 2,3-dichloro-nitrobenzène par nitration du 1,2-dichlorobenzène avec de l'acide nitrique, caractérisé en ce que, pour la nitration, on met en oeuvre un mélange anhydre d'acide phosphorique, d'acide sulfurique et d'acide nitrique, un des acides étant mis en oeuvre avec une fraction d'anhydride qui est appropriée pour consommer la fraction minime d'eau des deux autres acides.

2. Procédé selon la revendication 1, caractérisé en ce qu'on met en oeuvre, outre de l'acide nitrique approximativement anhydre et de l'acide sulfurique approximativement anhydre, un acide phosphorique dont la concentration en P₂O₅ est supérieure à 72,4%.

3. Procédé selon la revendication 1, caractérisé en ce que la proportion molaire de l'acide sulfurique et de l'acide phosphorique prend des valeurs de 0,05 à 3 moles de H₂SO₄:1 mole de H₃PO₄.

4. Procédé selon la revendication 1, caractérisé en ce qu'on effectue la nitration à une température réactionnelle de 30 à 180°C.

5. Procédé selon la revendication 1, caractérisé en ce qu'on met en oeuvre l'acide nitrique dans un rapport molaire de 0,7 à 1,4.

6. Procédé selon la revendication 4, caractérisé en ce que, pour augmenter la fraction du 2,3-dichloro-nitrobenzène par rapport à celle du 3,4-dichloro-nitrobenzène, on travaille dans la partie supérieure du domaine de température.

7. Procédé selon la revendication 1, caractérisé en ce qu'on libère de l'eau par distillation le mélange d'acides obtenu lors du traitement du produit et on le recycle.
